# EUROPEAN PATENT SPECIFICATION

(11) **EP 3 294 187 B1**
(45) Date of publication and mention of the grant of the patent: **12.09.2018**
(21) Application number: 17758438.0
(22) Date of filing: 28.07.2017
(51) Int. Cl.: A61B 90/50, A61B 34/30

(54) **MEDICAL INSTRUMENT HOLDER**
MEDIZINISCHES INSTRUMENTENHALTER
SUPPORT D'INSTRUMENT MEDICAL

(30) Priority: 28.07.2016 EP 16181765
(43) Date of publication of application: 21.03.2018
(73) Proprietor: Thys, Tom, 3200 Aarschot (BE); Thys, André, 3200 Aarschot (BE); Thys, Andy, 3200 Aarschot (BE)
(72) Inventor: Thys, Tom, 3200 Aarschot (BE); Thys, André, 3200 Aarschot (BE); Thys, Andy, 3200 Aarschot (BE)
(74) Representative: LC Patents
(86) International application number: PCT/EP2017/069205
(87) International publication number: WO 2018/020018

(56) References cited:
- US-A1- 2012 132 018
- US-A1- 2013 123 798
- US-A1- 2013 331 644
- US-B1- 6 406 472
- Chin-Hsing Kuo, Jian S Dai: "Robotics for Minimally Invasive Surgery: A Historical Review from the Perspective of Kinematics" In: "International Symposium on History of Machines and Mechanisms", 1 January 2009 (2009-01-01), Springer Netherlands, XP002766009, ISBN: 978-1-4020-9484-2 pages 343-351, DOI: 10.1007/978-1-4020-9485-9_24, figure 20b page 351

## Description

### FIELD OF THE INVENTION

The present invention is directed to a holder which has to be combined with an external object whereby the external object moves around a remote centre of motion. The external object preferably is a medical instrument such as a cannula or trocar whereby the remote centre of motion is the incision in the patient's abdomen. The holder can be used to support another object, in a particular embodiment a camera, convenient for performing endoscopic surgery, including in video-assisted thoracoscopic surgery, laparoscopic surgery and arthroscopic surgery; and is equipped with means for generating motion, both in a longitudinal and rotational manner following XYZ coordinates. The holder of the present invention is particularly useful for working in small areas as it occupies only a small bedside space, without sacrificing on the functional work area of the instrument mounted in the holder.

### BACKGROUND TO THE INVENTION

In standard laparoscopic abdominal surgery, incisions are made into the abdomen, followed by insufflation of the patient's abdomen with gas, and passing through cannulas via the small (approximately 1/2 inch) incisions to provide entry ports for laparoscopic surgical instruments. The laparoscopic surgical instruments generally include a laparoscope for viewing the surgical field, and working tools such as clamps, graspers, scissors, staplers, and needle holders. The working tools are similar to those used in conventional (open) surgery, except that the working tools are often thin and long with at one end tools working in the surgical field and at the other end handles manipulated by a surgeon. To perform surgical procedures, the surgeon passes instruments through the cannulas and manipulates them inside the abdomen by sliding them in and out through the cannulas, rotating them in the cannulas, and "levering" (pivoting) them around the centres of rotation approximately defined by the incisions in the muscles of the abdominal wall. This point is generally referred to as the Remote Centre of Motion (RCM). To maintain accurate positional control of an instrument during surgery, the surgeon may need to manually constrain it to pivot around the RCM coincident with the incision. Manual support of the pivot point is particularly of importance when the surgeon employs laparoscopes or other heavy instruments. Mechanical clamping devices are used to support the instruments in fixed orientations, but these devices do not provide a remote centre of rotation for positioning the instruments around the RCM.

Consequently, different RCM positioners have been developed relying on varying approaches to provide a remote centre of rotation and assist surgeons in minimal invasive surgery. The present approaches in guiding the instruments around the RCM can be categorized according to the kinematic mechanism used. One of the best-known mechanisms is the one employed in the RCM arm of the daVinci® surgical system (US 7,108,688) and relies on a double-parallelogram to constrain a surgical instrument to move around a fixed centre of rotation. The major disadvantage of this double-parallelogram mechanism is that it requires a large amount of working space above the patient.

Other systems employ circular tracking arcs (such as the EndoBot of Rensselaer Polytechnic institute), a fixed isocenter (such as the CLEM of Institut Albert Bonniot), a spherical linkage mechanisms (such as the RAVEN of Univ. of Washington), a highly complex gear train (such as the CoBRASurge of Univ. of Nebraska-Lincoln), a synchronous belt system (such as the MicroHand A of Tianjin University) or a set of parallel linkages pivotably connected to a gripper at fixed distances from the remote centre of motion (such as the VESALIUS system described in US2012/0132018). Another robotic system, based on a planar remote center of motion device is described in US2013/123798, as described in the review article of Kuo & Dai, 2009 "Robotics for Minimally Invasive Surgery: A Historical Review from the Perspective of Kinematics", in "International Symposium on History of Machines and Mechanisms", Springer Netherlands, ISBN: 978-1-4020-9484-2; pages 343-351.

It has been an objective of the present invention to provide an instrument holder which does not necessarily needs to have a fixed remote centre of motion but has to be combined with an external object (e.g. cannula, trocar, ...) whereby the external object moves around a RCM (fixed or not), in particular during video-assisted thoracoscopic surgery, laparoscopic surgery, and arthroscopic surgery; more in particular laparoscopic surgery. In great contrast with US2013/331644, describing an intelligent autonomous camera control for robotics with medical, military and space applications, requiring multiple members with a multitude of coupling types between said members to enable full spatial positioning of the camera, the instrument holder of the present is much simpler in design with only two base members to allow the external object to move around a RCM. Compared to the system described in US2013/331644, the present invention differs that the first member is not only configured to allow a longitudinal displacement of its connection with the second member but is equally configured to allow rotation of the first member around its longitudinal axis. In US2013/331644, a further shoulder member is required instead. This not only has an impact on the space occupied by the instrument holder, but also means that more complex couplings are required between said shoulder member and the further elbow member present in US2013/331644. No such further intermediate shoulder member is required in the instrument holder of the present invention, significantly reducing the space occupied and by relying on a first member configured to allow both a longitudinal and rotary motion of its connection with the second member along its longitudinal axis, the further design is greatly simplified without loss of functionality.

US 6,406,472 discloses a medical instrument holder which has to be combined with an external object that moves around a remote centre of motion, having a first member oriented in line with an axis with its origin at the remote centre of motion of said object, having a second member which is connected by means of a pivotable connector to the first member at one end and wherein the first member is configured to allow a circumferential displacement of the pivotable connector along said axis of the first member.

### SUMMARY OF THE INVENTION

In general, the goal of the present invention is to provide medical instrument holder for an object, in a particular embodiment a camera, during laparoscopic surgery or other high precision surgeries, i.e. minimal invasive surgical procedures such as video-assisted thoracoscopic surgery, and arthroscopic surgery, that has to be combined with another object, in a particular embodiment a cannula or trocar that moves around a remote centre of motion (the incision in the patient, for example the incision in the patient's abdomen in case of laparoscopic surgery). Expressed differently, the external object is held at an isocenter (its remote centre of motion) and connected to the holder wherein the holder is configured to follow the motion of the external object around the isocenter (around the remote centre of motion). The RCM may be a fixed or non-fixed RCM.

In a first aspect, the present invention provides a medical instrument holder, said holder having a first member oriented in line with an axis, for example the X-axis of the XYZ coordinate, with its origin at the remote centre of motion. The holder further comprises a second member, which is connected at one end on a pivotable manner (pivotable connector) to the first member and having coupling means at the other end for connecting a certain object (the external object) that is able to move around a remote centre of motion, preferably a medical instrument such as a cannula or trocar; Said coupling means may be flexible, wherein said coupling means permit rotation in at least two planes and wherein said coupling means are displaced laterally relative to said X-axis by means of said second member. Furthermore, the first member of the holder is configured to allow a longitudinal displacement of the pivotable connector along said axis. Expressed differently, the holder comprises; - a first member oriented in line with an axis, for example the X-axis of the XYZ coordinate, with its origin at the remote centre of motion; - a second member, which is connected at one end on a pivotable manner (pivotable connector) to the first member and having a flexible coupling at the other end for connecting a certain object (the external object) that is able to move around a remote centre of motion; characterized in that the first member is configured to allow a longitudinal displacement of the pivotable connector along said axis; and to allow said pivotable connector to rotate about said axis.

Per reference to the exemplified embodiments hereinafter, in one embodiment the longitudinal displacement and the circumferential displacement of the pivotable connector along the longitudinal axis of the first member is realized by a fixed position of the pivotable connector at the first member and configuration elements allowing a longitudinal displacement of the first member along its longitudinal axis and allowing rotation of the first member along its longitudinal axis. In another embodiment the longitudinal displacement and the circumferential displacement of the pivotable connector along the longitudinal axis of the first member is realized by a slidable position of the pivotable connector at the first member and configuration elements in the first member including guides for the pivotable connector along its longitudinal axis and configuration elements allowing rotation of the first member along its longitudinal axis.

In a particular embodiment the flexible coupling is exchangeable. In this embodiment the second member comprises an adaptor, enabling said flexible coupling to be placed at the free end of the second member.

Where the aforementioned configuration allows the holder to follow the motion of the external object around its remote centre of motion, it is a further object of the present invention to use the holder in securing the external object in a desired position. Hence in a further embodiment the holder comprises means to lock the moving members of the holder in-between the manipulation of the external object around its remote centre of motion. The moving members including the configuration elements of the first member to allow a longitudinal displacement of the pivotable connector along said X-axis and to allow said pivotable connector to rotate about said X-axis, wherein the configuration elements may allow a linear (3) and/or a concave-curved (4) displacement of the pivotable connection (2) along the longitudinal axis of the first member (100). The moving members further including the pivotable connection between the first and second member and eventually the flexible coupling between the second member and the external object. In one embodiment the holder comprises means to lock one or more of the moving members of the holder, said moving members being selected from the configuration elements of the first member to allow a longitudinal displacement of the pivotable connector along said X-axis; the configuration elements of the first member to allow rotation of the pivotable connector about said X-axis; the pivotable connection between the first and second member; and the flexible coupling between the second member and the external object. In a particular embodiment the medical instrument holder comprises means to lock the configuration elements of the first member to allow a longitudinal displacement of the pivotable connector along said X-axis; the configuration elements of the first member to allow rotation of the pivotable connector about said X-axis; and optionally the pivotable connection between the first and second member. In another particular embodiment the holder comprises means to lock the configuration elements of the first member to allow a longitudinal displacement of the pivotable connector along said X-axis; the configuration elements of the first member to allow rotation of the pivotable connector about said X-axis; the pivotable connection between the first and second member; and optionally the flexible coupling between the second member and the external object.

As will be apparent to the skilled artisan, the holder can be used passively, wherein the movement of the holder passively follows the manipulation of the external object around its isocenter. Alternatively, the holder is used actively and controls the movement of the external object around its isocenter. Accordingly, in a further example, not part of the invention, the device comprises means for generating motion, both in a longitudinal and rotational manner following XYZ coordinates, thereby holding a given object, such as a camera.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

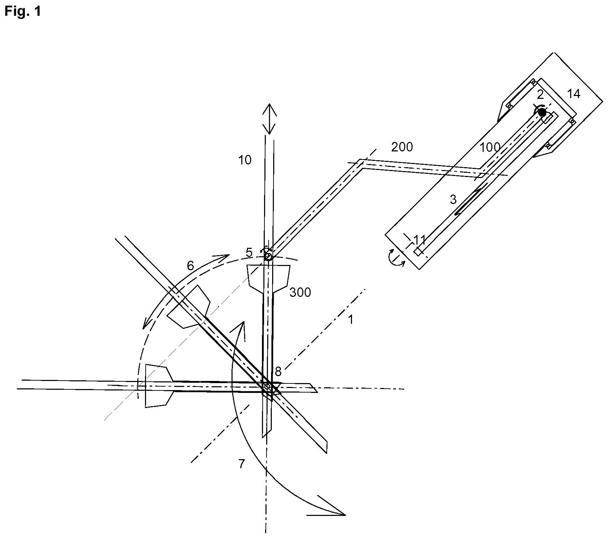

**Fig. 1****: Schematic overview of an embodiment of an instrument holder according to the invention.** Showing a first member (100) contained into a fixed body (14) with bearings, a second member (200) and the external object (300) (e.g. trocar, cannula, ...) coupled thereto and held spatially constrained at an isocenter (8). In this embodiment the flexible coupling means (5) and the external object (300) are fixed to each other. A laparascope (10) may be inserted into the external object (300).
   In this embodiment, the first member (100) is able to rotate within the fixed body (14) around its longitudinal axis, i.e. an X-axis (1) as herein represented. Furthermore, the pivotable connection (2) between the first (100) and second (200) member follows via a configuration element (linear guide (11)) in the first member (100) a longitudinal displacement along the X-axis (1), allowing a linear (3) displacement along the longitudinal axis of the first member (100). Being spatially constrained at an isocenter (8) the external object should be able to follow a spherical space around said isocenter (8), thus requiring the capability of a rotational movement about a first (indicated by arrow (6)) and a second (indicated by arrow (7)) direction.
   in the present embodiment of the instrument holder the rotational movement (6) is made possible by the movement caused by the pivotable connection (2) and the flexible coupling (5) together with the longitudinal displacement of the pivotable connection (2) in the linear guide (11), whereas rotational movement (7) is made possible because of the rotational movement of the first member (100) within the fixed body (14) along its longitudinal axis (1). Together these configuration elements enable movement of the flexible coupling according to rotational movement (6) and (7).

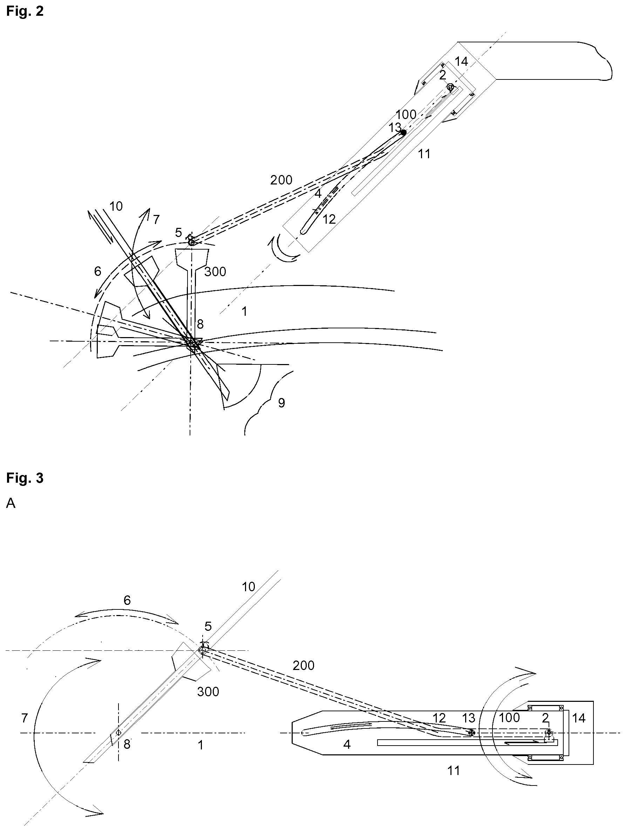

**Fig. 2****: Schematic overview of an embodiment of an instrument holder according to the invention.** Showing a first member (100) contained into a fixed body (14) with bearings, a second member (200) and the external object (300) (e.g. trocar, cannula, ...) coupled thereto and held spatially constrained at an isocenter (8). In this embodiment the connection (in particular a releasable connection) (5) is a flexible connection (e.g. ball joint). In this embodiment the flexible coupling (5) and the external object (300) are NOT fixed to each other. A laparascope (10) may be inserted into the external object (300), wherein the access area of the external object (300) is the organ (9) of the patient.
   In this embodiment, the first member (100) is able to rotate within the fixed body (14) around its longitudinal axis, e.g. an X-axis (1). Furthermore, the pivotable connection (2) between the first (100) and second (200) member follows via a first configuration element (linear guide (11)) in the first member (100) a longitudinal displacement along the X-axis (1), allowing a linear displacement (3) along the longitudinal axis of the first member (100). In addition, in this embodiment, the second member comprises a further guide connector (13) that fits in a second configuration element (12) (a concave -curved guide (12)) in the first member (100), and follows mainly a curved (4) displacement along the longitudinal axis of the first member (100).
   Hence,in this embodiment rotational movement (6) is made possible by the movement caused by the pivotable connection (2), the guide connector (13) and the flexible coupling (5), whereas rotational movement (7) is made possible because of the rotational movement of the first member (100) within the fixed body (14) along its longitudinal axis (1), thereby allowing and together with the foregoing configuration elements, movement of the flexible coupling according to rotational movement (6) and (7).

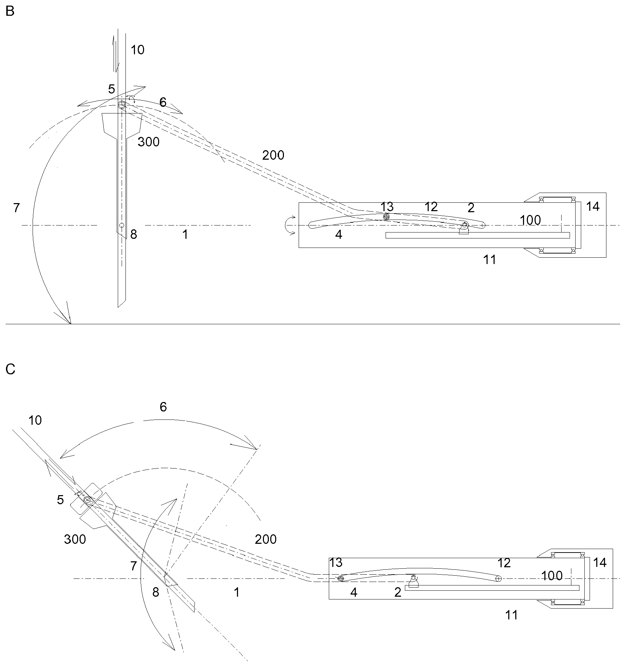

**Fig. 3****: Schematic overview, showing three positions (A, B and C) for an instrument holder according to the invention and caused by the longitudinal displacement of second member (200) along the configuration elements (11, 12) in the first member (100) of the holder. A** showing the fully retracted position. **B** showing an intermediate position. **C** showing a fully extended position.

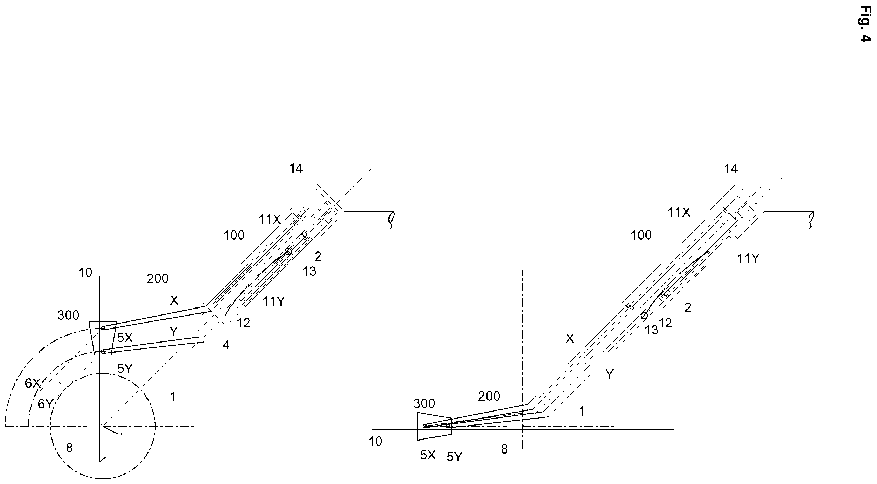

**Fig. 4****: Schematic overview of an embodiment of an instrument holder according to the invention.** Showing a first member (100) contained into a fixed body (14) with bearings, a second member (200) and the external object (300) (e.g. trocar, cannula, ...) coupled thereto and held spatially constrained at a fixed isocenter (8). In this embodiment, the second member (200) comprises two arms, an upper (X) and lower (Y) arm, each comprising a linear guide (11X), and (11Y) in the first member. The guide of the lower arm (11Y) even comprises a concave-curved guide (12) and a guide connector (13). Furthermore, each arm has its own coupling means (5X and 5Y), said coupling means being fixed together to the external object (300). A laparascope (10) may be inserted into the external object (300).
   In this embodiment, the first member (100) is able to rotate within the fixed body (14) around its longitudinal axis, i.e. an axis (1) crossing the isocenter (8) as herein represented. Furthermore, the pivotable connection (2) between the first (100) and second (200) member follows via a configuration element (linear guide (11Y)) in the first member (100) a longitudinal displacement along the X-axis (1). Furthermore, the upper arm X having flexible means near the fixed body (14) enforces the angle of the movement. Being spatially constrained at an isocenter (8) the external object should be able to follow a spherical space (front-, back-, sidewards, ..) around said isocenter (8), thus requiring the capability of a rotational movement about a first (indicated by arrow (6X and 6Y)) direction. In the present embodiment of the instrument holder the rotational movement (6X and 6Y) is made possible by the movement caused by the pivotable connection (2) and the coupling means (5X and 5Y) together with the longitudinal displacement of the pivotable connection (2) in the linear guides (11X and 11Y), During this action, the lower arm will follow the rotational movement, whereas the upper arm enables the generation of a fixed isocenter (8). Evidently, speed of both arms contained within the first member varies during movement as it is function of both the rotational movement and the distance between the upper and lower arms.

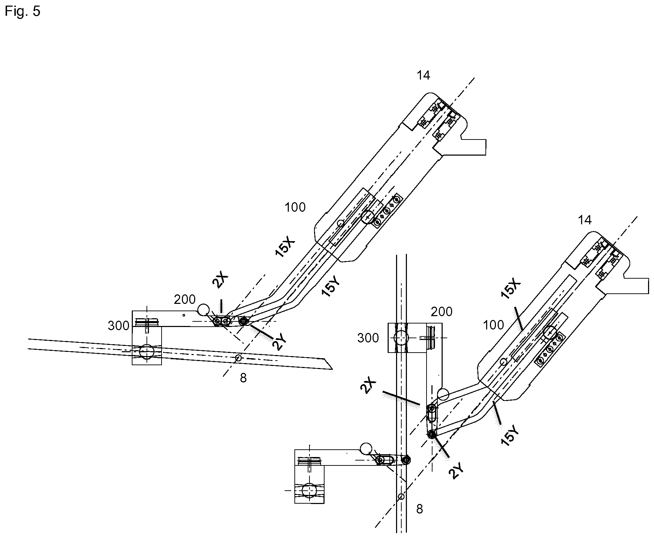

**Fig. 5****: Schematic overview of an alternative embodiment of an instrument holder according to the invention.** Showing a first member (100) contained into a fixed body (14) with bearings, a second member (200) and the external object (300) (e.g. trocar, cannula, ...) coupled thereto and held spatially constrained at an isocenter (8). In this embodiment the first member is configured to rotate along its longitudinal axis and comprises two arms (15X and 15Y) arranged at a distance, in parallel to each other and configured to allow a longitudinal displacement along the longitudinal axis of the first member at a fixed ratio with respect to the first member. Said first and second arm are pivotable coupled the second member at respective distances of the isocenter (2X and 2Y) at a ratio equal to the ratio of the translational displacement of the two arms vis-a-vis the first member, and characterized in that the external object is coupled to the second member by means of a flexible coupling (5).

### DETAILED DESCRIPTION OF THE INVENTION

The present invention provides a holder for an (external) object (300), in a particular embodiment a cannula or trocar for minimally invasive surgical procedures, said holder having two members. Fig 1 is a schematic overview of an embodiment of the holder. In this embodiment the holder in itself does not need to have a remote centre of motion, hence when to be used during minimally invasive surgical procedures it has to be combined with another object (300), in a particular embodiment a cannula or trocar that moves around a remote centre of motion (the incision in the patient) (8). The instrument holder of the instant application can be used during a minimally invasive surgical procedure selected from video-assisted thoracoscopic surgery, laparoscopic surgery, and arthroscopic surgery; more in particular laparoscopic surgery. In said last embodiment the remote centre of motion coincides with the incision in the patient's abdomen. Not being limited to laparoscopic surgery, various tools or instruments like cannulas and trocars having a remote centre of motion, like the narrow entry points in laparoscopic surgery or other high precision surgery, can be supported by this invention. In said embodiment wherein the object is a cannula or trocar, various tools, like a laparoscope (10) can be inserted into said cannula or trocar and accordingly held by the holder of the instant application.

As already outlined herein before, the holder of present invention comprises a first member (100) which may be contained within a fixed body (14), and a second member (200) pivotally connected to one another by means of a pivotable connector (2); the second member having a coupling (object coupling) (5) to connect the holder to an (external) object (300) having an isocenter (8), and the first member comprising moving members to allow the object coupling (5) to follow the motion of the external object around the isocenter.

Thereto the first member (100) is oriented roughly in line with the X-axis (1) of the XYZ coordinate with its origin at the remote centre of motion (8) of the external object (300). For this initial orientation of the first member along said X-axis, the holder is typically mounted in a tripod or mounting fixture in the proximity of the isocenter of the external object.
The second member (200) is connected at one end on a pivotable manner (pivotable connection) (2) to the first member (100) and has coupling means (5) at the other end for engaging an (external) object (300), a medical instrument like a cannula or trocar in particular. Furthermore, said coupling means may be flexible, thereby permitting rotation in at least two planes and a displacement laterally relative to said X-axis by means of said second member.

Furthermore, the first member (100) of the holder is configured to allow a longitudinal displacement of the pivotable connector (2) along the longitudinal axis of the first member and to allow said pivotable connector to rotate about said axis.

In a certain embodiment the longitudinal displacement and the circumferential displacement of the pivotable connector (2) along the longitudinal axis of the first member (100) is realized by a fixed position of the pivotable connector (2) at the first member (100) and configuration elements allowing a longitudinal displacement of the first member (100) along its longitudinal axis and allowing rotation of the first member (100) along its longitudinal axis.

In another embodiment the longitudinal displacement and the circumferential displacement of the pivotable connector (2) along the longitudinal axis of the first member (100) is realized by a slidable position of the pivotable connector (2) at the first member (100) and configuration elements in the first member (100) including guides for the pivotable connector (2) along its longitudinal axis and configuration elements allowing rotation of the first member (100) along its longitudinal axis. Depending on the shape of the guides in the first member, these guides may allow longitudinal displacement in a linear (11), concave-curved (12) manner or combination thereof (infra).

In a particular embodiment, the holder is equipped with means to lock the moving members of the holder in-between the manipulation of the external object around its remote centre of motion (8), such as for example with friction couplings for the pivot point (2) between the first (100) and second (200) member, as well as for the configuration elements (e.g. guides) of the first member (100) allowing for the longitudinal and rotational displacement of the pivotable connector (2) along the X-axis (1). Once the orientation of the first (100) and second (200) member have been chosen according to the wishes of the person operating the instrument holder, these friction couplings can be locked using a manipulator. In a particular embodiment, the manipulator uses air pressure to lock the friction couplings. In said instances where the external object receives a further instrument (such as for example a laparoscope (10) inserted into a trocar) the former may comprise releasable engaging means to retain said further instrument within the external object in a desired position. In said embodiment, these releasable engaging means of the external object could equally consist of a friction coupling and in a preferred embodiment is being controlled by the same manipulator as the one used to release and engage the friction couplings present on the holder. As such, when releasing the friction components, the manipulator has full control in positioning the further instrument around the centre of motion of the external object (300) coupled to the holder of the instant application. To enhance the employability of the holder, in another embodiment the flexible coupling (5) is exchangeable. In this embodiment the second member comprises an adaptor, enabling said flexible coupling to be placed at the free end of the second member.

Furthermore, in a certain embodiment, the pivotable connection (2) between the first (100) and second (200) member follows in its longitudinal displacement along the X-axis (1) a linear displacement, which is the result of a linear configuration element, namely a linear guide (11) in the first member (100) of the holder. In another embodiment, the pivot point (2) connecting the second member to the first member does not merely follow a straight line along the X-axis of the XYZ coordinate with its origin at the remote centre of motion of said object (300), when being longitudinally displaced along said axis. In a particular embodiment the longitudinal displacement of said point includes a concave curved (4) displacement, wherein the turning point of the concave is oriented away from said X-axis. In this embodiment, the second member (200) comprises besides the first linearly displaced pivotable connection (2), a further guide connector (13) that fits in a second configuration element (12) (a concave - curved guide (12)) in the first member, and follows mainly a curved (4) displacement along the longitudinal axis of the first member. This embodiment results in a more constrained movement of the holder compared to the embodiment having only a linear displacement and makes it easier for the manipulator to make a more spherical rotation (front-, back-, sidewards) of the object coupling (5) connecting the external object (300) to the second member (200) around the remote centre of motion (8) of the external object. In a particular embodiment, the guide connector (13) and the pivotable connection (2) are the same.

Finally, the holder is made of sustainable materials, which in a certain embodiment are heat stable and therefore suitable for heat sterilisation.

To summarize, the present invention provides medical instrument a holder, particularly useful as an instrument (camera) holder in minimally invasive surgical procedures and differs from the current RCM manipulators in that the holder in itself does not need to have an RCM. The RCM is present in the object carried by the holder instead (herein also referred to as the external object). In a preferred embodiment this external object is a trocar for laparoscopic or video-assisted thoracoscopic surgery and inserted into the incision of the patient's abdomen or thorax. When inserted this trocar is spatially constraint at said incision point, making this point to behave as a remote centre of motion for the trocar. Coupling the object to the holder of the instant application does not further constrain the manipulation of the object. The holder accordingly provides a compact solution to keep a laparoscope or endoscope at a desired orientation, without making concessions on the operational area of manipulation. This large operational area of manipulation is for example apparent in figure 2 showing the access area of the external object (300), in said example of a trocar, to the organ (9) in the patient.

## Claims

1. A medical instrument holder
- which has to be combined with an external object (300) that moves around a remote centre of motion (8); and
- having a first member (100) oriented in line with an axis (1) with its origin at the remote centre of motion (8) of said object (300);
- having a second member (200) which is connected by means of a pivotable connector (2) to the first member (100) at one end and has coupling means (5) for holding said external object (300) at the other end; and wherein
- the first member (100) is configured to allow a longitudinal and circumferential displacement of the pivotable connector (2) along said axis (1) of the first member (100), to allow said external object (300) to move around said remote centre of motion (8).

2. The holder of claim 1, wherein the longitudinal displacement and circumferential displacement of the pivotable connector (2) along said axis (1) of the first member (100) is realized by a fixed position of the pivotable connector at the first member and configuration elements allowing a longitudinal displacement of the first member along its longitudinal axis and allowing rotation of the first member along its longitudinal axis.

3. The holder of claim 1, wherein the longitudinal displacement and the circumferential displacement of the pivotable connector (2) along said axis (1) of the first member (100) is realized by a slidable position of the pivotable connector (2) at the first member (100) and configuration elements allowing rotation of the first member along its longitudinal axis.

4. The holder according to any one of claims 1 to 3, wherein the longitudinal displacement of the pivotable connector (2) is linear.

5. The holder of claim 3, wherein the first member (100) is equipped with a linear guide (11) for the slidable position of the pivotable connector (2).

6. The holder according to claim 3, wherein the longitudinal displacement of the pivotable connector (2) includes a concave curved (4) displacement, wherein the turning point of the concave is oriented away from said axis (1).

7. The holder of claim 6, wherein the second member (200) comprises a further guide connector (13).

8. The holder of claims 6 or 7, wherein the first member (100) is equipped with a linear guide (11), a concave-curved guide (12) and a guide connector (13)

9. The holder of claim 1, wherein said coupling means (5) are flexible.

10. The holder of claim 9, wherein said coupling means permit rotation in at least two planes.

11. The holder of claim 10, wherein said coupling means (5) are displaced laterally relative to said axis (1) by means of said second member (200).

12. The holder of claim 9 to 11, wherein said coupling means (5) are exchangeable.

13. The holder of claim 1, where the external object (300) is a medical instrument or medical tool.

14. The holder according to previous claims, where said holder is equipped with means to lock the first (100) and /or the second member (200).

## Patentansprüche

1. Ein medizinischer Gerätehalter,
- der mit einem externen Objekt (300), das sich um das Remote-Bewegungszentrum (8) bewegt, kombiniert werden muss; und
- mit einem ersten Element (100), das in einer Linie mit einer Achse (1) ausgerichtet ist, deren Ursprung im Remote-Bewegungszentrum (8) des genannten Objekts (300) liegt;
- mit einem zweiten Element (200), das mittels eines schwenkbaren Konnektors (2) mit dem ersten Element (100) an einem Ende verbunden ist und Verbindungsmitteln (5) zum Halten des genannten externen Objekts (300) am anderen Ende hat; und wobei
- das erste Element (100) konfiguriert ist, um eine Längs- und Umfangsverschiebung des schwenkbaren Konnektors (2) entlang der genannten Achse (1) des ersten Elements (100) zu ermöglichen, damit sich das genannte externe Objekt (300) um das genannte Remote-Bewegungszentrum (8) bewegen kann.

2. Der Halter nach Anspruch 1, wobei die Längs- und Umfangsverschiebung des schwenkbaren Konnektors (2) entlang der genannten Achse (1) des ersten Elements (100) durch eine feste Position des schwenkbaren Konnektors am ersten Element und den Konfigurationselementen, die eine Längsverschiebung des ersten Elements entlang seiner Längsachse und eine Drehung des ersten Elements entlang seiner Längsachse ermöglichen, realisiert wird.

3. Der Halter nach Anspruch 1, wobei die Längs- und Umfangsverschiebung des schwenkbaren Konnektors (2) entlang der genannten Achse (1) des ersten Elements (100) durch eine verschiebbare Position des schwenkbaren Konnektors (2) am ersten Element (100) und den Konfigurationselementen, die eine Drehung des ersten Elements entlang seiner Längsachse ermöglichen, realisiert wird.

4. Der Halter entsprechend einem der Ansprüche 1 bis 3, wobei die Längsverschiebung des schwenkbaren Konnektors (2) linear ist.

5. Der Halter nach Anspruch 3, wobei das erste Element (100) mit einer Linearführung (11) für die verschiebbare Position des schwenkbaren Konnektors (2) ausgestattet ist.

6. Der Halter nach Anspruch 3, wobei die Längsverschiebung des schwenkbaren Konnektors (2) eine konkav gekrümmte (4) Verschiebung aufweist, wobei der konkave Wendepunkt von der genannten Achse (1) weggerichtet ist.

7. Der Halter nach Anspruch 6, wobei das zweite Element (200) einen weiteren Führungskonnektor (13) aufweist.

8. Der Halter nach Anspruch 6 oder 7, wobei das erste Element (100) mit einer Linearführung (11), einer konkav gekrümmten Führung (12) und einem Führungskonnektor (13) ausgestattet ist.

9. Der Halter nach Anspruch 1, wobei die genannten Verbindungsmittel (5) flexibel sind.

10. Der Halter nach Anspruch 9, wobei die genannten Verbindungsmittel eine Drehung in mindestens zwei Ebenen ermöglichen.

11. Der Halter nach Anspruch 10, wobei die genannten Verbindungsmittel (5) durch das genannte zweite Element (200) seitlich zur genannten Achse (1) verschoben sind.

12. Die Halter der Ansprüche 9 bis 11, wobei die genannten Verbindungsmittel (5) austauschbar sind.

13. Der Halter nach Anspruch 1, wobei das externe Objekt (300) ein medizinisches Gerät oder ein medizinisches Werkzeug ist.

14. Der Halter entsprechend den vorhergehenden Ansprüchen, wobei der Halter mit Mitteln ausgestattet ist, um das erste (100) und/oder das zweite Element (200) zu verriegeln.

## Revendications

1. Un support d'instrument médical
- qui doit être combiné avec un objet externe (300) qui pivote autour d'un centre de rotation déporté (8) ; et
- ayant un premier membre (100) aligné sur un axe (1) ayant son origine au centre de rotation déporté (8) dudit objet (300) ;
- ayant un second membre (200) qui est relié par un connecteur pivotant (2) au premier membre (100) à une extrémité et possède un moyen de couplage (5) pour maintenir ledit objet externe (300) à l'autre extrémité ; et dans lequel
- le premier membre (100) est configuré pour permettre un déplacement longitudinal et circonférentiel du connecteur pivotant (2) le long dudit axe (1) du premier membre (100), pour permettre audit objet externe (300) de pivoter autour dudit centre de rotation déporté (8).

2. Le support selon la revendication 1, dans lequel le déplacement longitudinal et le déplacement circonférentiel du connecteur pivotant (2) le long dudit axe (1) du premier membre (100) sont réalisés par une position fixe du connecteur pivotant sur le premier membre et les éléments de configuration permettant un déplacement longitudinal du premier membre le long de son axe longitudinal et permettant la rotation du premier membre le long de son axe longitudinal.

3. Le support selon la revendication 1, dans lequel le déplacement longitudinal et le déplacement circonférentiel du connecteur pivotant (2) le long dudit axe (1) du premier membre (100) sont réalisés par une position coulissante du connecteur pivotant (2) sur le premier membre (100) et les éléments de configuration permettant la rotation du premier membre le long de son axe longitudinal.

4. Le support selon l'une des revendications 1 à 3, dans lequel le déplacement longitudinal du connecteur pivotant (2) est linéaire.

5. Le support selon la revendication 3, dans lequel le premier membre (100) est équipé d'un guide linéaire (11) pour la position coulissante du connecteur pivotant (2).

6. Le support selon la revendication 3, dans lequel le déplacement longitudinal du connecteur pivotant (2) inclut un déplacement concave (4), dans lequel le point tournant du concave est orienté loin dudit axe (1).

7. Le support selon la revendication 6, dans lequel le second membre (200) comprend un autre connecteur de guide (13).

8. Le support selon la revendication 6 ou 7, dans lequel le premier membre (100) est équipé d'un guide linéaire (11), d'un guide incurvé à face concave (12) et d'un connecteur de guide (13).

9. Le support selon la revendication 1, dans lequel les moyens de couplage (5) sont flexibles.

10. Le support selon la revendication 9, dans lequel les moyens de couplage (5) permettent une rotation dans au moins deux plans.

11. Le support selon la revendication 10, dans lequel lesdits moyens de couplage (5) sont déplacés latéralement par rapport audit axe (1) au moyen dudit second membre (200).

12. Le support selon les revendications 9 à 11, dans lequel les moyens de couplage (5) sont échangeables.

13. Le support selon la revendication 1, où l'objet externe (300) est un instrument médical ou un outil médical.

14. Le support selon les revendications précédentes, où ledit support est équipé de moyens destinés à verrouiller le premier (100) et/ou le second membre (200).
